# EUROPEAN PATENT SPECIFICATION

(11) **EP 0 574 547 B1**
(45) Date of publication and mention of the grant of the patent: **04.10.2001**
(21) Application number: 92910571.6
(22) Date of filing: 03.03.1992
(51) Int. Cl.: A61L 9/12, A61L 9/14, B05B 5/025

(54) **ELECTROSTATIC VAPOR-AEROSOL GENERATOR**
ELEKTROSTATISCHER DÄMPFE-AEROSOLEERZEUGER
GENERATEUR DE VAPEURS ET/OU D'AEROSOLS ELECTROSTATIQUE

(30) Priority: 11.03.1991 US 667200
(43) Date of publication of application: 22.12.1993
(73) Proprietor: In-Vironmental Integrity, Inc., Minneapolis, MN 55416 (US)
(72) Inventor: Peltier, Mark E., Minneapolis, MN 55416 (US)
(74) Representative: Lowther, Deborah Jane
(86) International application number: PCT/US92/01812
(87) International publication number: WO 92/15339

(56) References cited:
- EP-A- 0 120 633
- EP-A- 0 486 198
- FR-A- 1 069 841
- GB-A- 2 233 230
- US-A- 2 140 516
- US-A- 4 776 515

## Description

This invention in general is intended to generate vapor and/or aerosols from a liquid by the application of an electrostatic charge to a porous, preferably semi-conductive, capillament assembly which receives the liquid. More specifically a high voltage DC or AC signal is introduced to a device referred to herein as an "electrostatic wick" or a "vaporizing emitter." This invention has a particular application in the vaporization of essential oils commonly used in the fragrance industry. Essential oils derived from plants, trees, and flowers; also perfumes, natural and synthetic; deodorants, disinfectants, fumigants, fungicides, insecticides, and other liquid substances either water or hydrocarbon based which may be intended to modify, condition, or alter the quality of an indoor or outdoor atmosphere can be vaporized more effectively using such apparatus.

There is a growing concern in the area of indoor air quality often referred to as "sick building syndrome." The modern living and working environment has been designed around energy efficiency and not so oriented around occupant health and comfort. It would be desirable to be able to recreate the properties of fresh outdoor air, indoors. The existence of air ions and their benefit is well documented. Over the last five years there has also been an increased interest in aromatic essences from plants and their application to enhancing or altering the quality of an indoor environment.

The vaporization of aromatic essences and other liquids and also the generation of negative ions is the basis for this invention. The original embodiment of this invention is based upon the need to enhance indoor air quality. This invention can also be modified for applications where it is desirable to generate vapor or aerosols using electrostatic means.

The vaporization of liquids is accomplished by a variety of apparatus and there are also many devices which use electrostatic means to generate aerosols. This invention is specific to the generation of vapor and/or aerosols more efficiently in a range of sizes from a variety of liquids with more control than the prior art, and it can also generate air ions.

One prior art apparatus for generating a mist of negatively charged liquid aerosols is described in U.S. Patent No. 4,776,515 to Michalchik. The limitations of the patent based on the claims are that a very specific conductivity of the liquid is required and that charged particles are generated, not a vapor. The device also has specific requirements upon the manner in which the liquid is fed to the capillary in order to maintain the desired aerosol generating effect.

An apparatus for producing a spray of liquid droplets of a specific size range is covered in U.S. Patent No. 4,829,996. This device is specific to the production of particles by electrostatic means of a certain size and specifically not a vapor. This device is specifically an electrostatic spray generator for an inhaler.

The electrostatic dispersal of liquids by Pollard et al in U.S. Patent No. 4,400,332 is described with particular application for dispersing petrol into an air stream for combustion in an internal combustion engine. That document describes the use of a porous material having a series of termini which is fed the liquid, namely, petrol fuels. This porous material is charged electrostatically and a spray of fine particles are formed in an air stream. This device produces very fine particles within an air stream wherein an annular enclosure is required. Here again vapor is not mentioned and a moving air steam is involved.

EP-A-0486198 (filed 4.11.91, published 20.05.92) describes spraying apparatus in which a liquid, such as a fragrance-producing oil, is sprayed from a reservoir via a vertically disposed capillary structure, an electrical potential being applied to the liquid so it is drawn across the end face of the capillary structure and sprayed as ligaments which break up into droplets.

Electrostatic enhancement of evaporation by French et al in U.S. Patent No. 3,771,233 covers a method of specifically improving the evaporation of water from investment cast ceramic molds using an electrostatic charge placed upon the mold. The evaporation process is enhanced with a series of needles of an opposite charge placed near the surface of the mold. This method is specific in claim to the evaporation of water from investment castings. In this case evaporating water is the only objective.

This invention is an improvement upon these methods and others such that both vapor and/or aerosols can be generated from the same device. Another advantage is that the rate of vapor generation can be controlled by the adjustment of the voltage applied to the "emitter", and/or the liquid feed rate and/or the placement of an electrostatic field forming control grid near the emitter. An additional advantage is that various sizes of aerosols can be generated from the same emitter by simple adjustment of these field forming control grids. This invention also will generate air ions of the same polarity as the supply voltage.

The present invention provides an apparatus for generating electrostatically charged aerosols and/or vapors comprising: a porous, capillary unit of elongated configuration having a dispensing end; said capillary unit being comprised of a plurality of fiber filaments arranged in a tubular configuration which terminate at said dispensing end in a plurality of exposed, freely extending dispensing tips; electrode means, said electrode means comprising an electrode in contact with said capillary unit; electrical contact means on said electrode for connecting said electrode to a source of high voltage, direct current power; and means, for supplying liquid to said capillary unit, whereby liquid can pass through said porous capillary unit in proximity to said electrode and be electrostatically charged and dispensed as aerosols and/or vapors from said freely extending tips of said capillary unit.

There is further provided a method of generating air quality enhancing vapors and/or aerosols and dispensing them into the air handling system of a building comprising: placing an emitter having a dispensing end in air flow communication with the air handling system of a building, said emitter comprising: a plurality of fiber filaments arranged in a tubular configuration which terminate at said dispensing end in a plurality of exposed, freely extending dispensing tips; liquid passage means; means for supplying liquid to said liquid passage means; means for imparting an electrostatic charge to liquid in said emitter; and, air flow passage means in fluid flow juxtaposition to said liquid passage means; directing a pressurized flow of liquid to said liquid passage means from said means for supplying liquid, said liquid being selected from the group of air quality enhancing substances comprising aromatic essences, deodorants, disinfectants, fumigants, fungicides, insecticides, and bactericides; applying an electrostatic charge to liquid in said emitter and thereby generating vapors and/or aerosols from said freely extending tips; and, passing a stream of pressurized air through said air flow passage means and thereby picking up said electrostatically charged vapors and/or aerosols in said air stream; and thence directing said stream of pressurized air into said air handling system.

The concept of electrostatic vaporization was conceived in an attempt to disperse a vapor of a conditioning substance and also generate negative air ions into the air of a building to improve indoor air quality. A typical embodiment of the invention will be comprised of, but not limited to, the following components.

A high voltage DC power supply with an adjustable output (5-35 kilovolts negative) is used to power an "electrostatic wick" assembly which is comprised of a central conductive electrode, an outer porous capillary material, and a vial, vessel, or tubular enclosure used to contain and direct the liquid to be vaporized. If the liquid is supplied to the apparatus by the use of a tube or pipe and if there is no requirement to "wick" the liquid, then the device is referred to as a "vaporizing emitter". In both devices the main components of the wick or emitter would be summarized as an electrostatically charged, liquid-fed, preferably semi-conductive, porous, capillary assembly. The capillary assembly must comprise a plurality of fiber filaments arranged in a tubular configuration which terminate at the dispensing end in a plurality of exposed freely extending dispensing tips.

These "wicks" and "emitters" were fabricated from the following materials in hundreds of combinations in order to obtain the best vapor/aerosol generation performance for the test liquid and also the optimum air ionization output.

Conductive foam, ceramic fibers, graphite fibers, porous ceramic, porous polyethylene foam, porous sintered metals (discs, tubes, spheres, and sheets of stainless steel and brass), glass wool, Fiberglas braiding, graphite braiding, stainless steel braiding, glass tubing, polycarbonate tubing, wool wicking, wool felts, and other materials were used alone and in combination.

In most cases the most efficient "wicks or emitters" for all liquids tested were fabricated from a combination of a conductive center electrode and an outer semi-conductive or nonconductive porous capillary material.

These charged "wicks" or emitters" directly effect the natural vapor pressure of any liquid which is applied to them at any given temperature and atmospheric pressure by using electrostatic forces acting upon the surface tension of the liquid held within a porous mass or wicking assembly.

The initial objective of this invention was to efficiently vaporize an aromatic essential oil into an office environment. After finding the use of an electrostatic charge applied to a conductive porous mass or wick a highly effective vaporization system, it is necessary to outline additional objectives of the invention.

The principal object of the present invention is to generate electrostatically charged vapors and aerosols from a liquid using high voltage direct current (DC) which is applied to a vaporizing emitter or wick assembly.

An additional object of this invention is to be able to precisely control the rate of generation of vapor, and/or aerosols by controlling the voltage applied and thereby the electrostatic charge upon the emitter or wick, the electric field pattern and also the volume rate of liquid feed to the wick or emitter.

Another object of this invention is to electrostatically charge the vapors and/or aerosols of the liquid to a desired polarity and through the use of electrostatic fields, control the shape and pattern of the vapor/aerosols that are generated.

An additional object of this invention is to release these charged vapors and aerosols directly into the air of a room, or onto the inner surfaces of ventilation system duct work of a building or onto the surfaces of ventilation system mechanical equipment and/or to distribute the vapor/aerosols throughout a building through the ventilation system.

The final object of this invention is to select specific liquid chemical formulations which in vapor phase and/or aerosol form may be electrostatically charged, will have properties such that when they are introduced into the air of a room or a building ventilation system they will modify the character and quality of the air by adding natural aromas, synthetic scents or combinations which may also include disinfection agents, fungicides, bactericides, viruscides and related formulations which could be used to disinfect building ventilation duct work and related ventilation system equipment.

Following the above mentioned introduction to the concept, advantages and the objectives of this invention, preferred embodiments of the present invention will now be described in detail by way of example only, with reference to the following drawings, in which:

Fig. 1 shows the most basic embodiment of the concept of the use of electrostatic charges to vaporize a liquid directly from a porous mass or wick. Also shown are the basic components in exploded view.

Fig. 2 is a perspective view of a preferred embodiment of a "wick" assembly partially in section.

Fig. 3 shows the installation of the "wick" assembly shown in Fig. 2 within a glass bottle. This embodiment is a self-contained liquid storage and vapor/aerosol dispensing device.

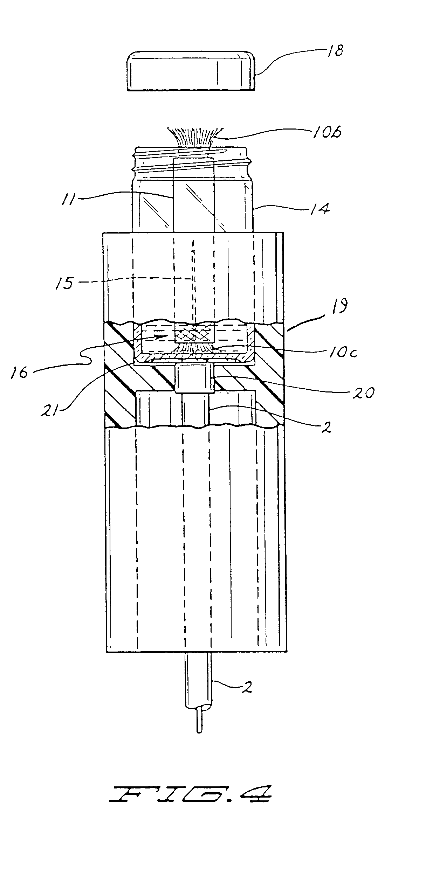

Fig. 4 is an illustration of an embodiment of a means to hold the device of Fig. 3 and apply an electrostatic charge to it.

Fig. 5 is an embodiment of a "vaporizing emitter" for use within a moving air stream. This model requires a controlled liquid feed to the emitter in addition to the electrostatic charge.

Fig. 6 is an illustration of another vaporizing emitter which also requires a controlled liquid feed and a moving air stream and is intended for building duct work applications (not part of the invention). In consideration of the drawings submitted and the partial list of materials used to construct these vaporizing emitters, it is not possible to illustrate every combination. These serve as examples that have proven to be effective under test and have demonstrated the concept of the electrostatic vaporization of selected liquids.

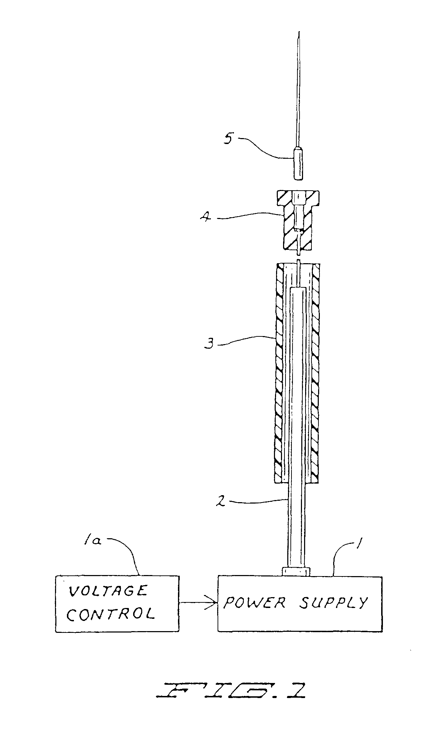

Fig. 1 illustrates a basic embodiment of the power source, as an exploded view. A high voltage DC power supply 1 with an adjustable output 1a (5-35 kilovolts negative 200 microamps) supplies power to a terminal 4 via a high voltage wire conduit 2. An electrode 5 is inserted in the terminal and provides the charge to a wick assembly. High voltage terminal 4 is inserted into a polycarbonate tube 3 as a support.

The electrode is inserted into the wick material or assembly, the desired liquid is supplied to the wick and power is applied at the desired voltage. The properties of the materials that comprise the wick have a significant effect upon the vapor and/or aerosol output efficiency at any given voltage setting. The porosity, conductance, and shape of the materials will determine the vapor-aerosol ratio and also the amount of air ions generated.

Where the wick is fabricated from a nonconductive porous material, the electrode is preferably inserted into the wick so that the entire electrode is covered by the wick. In this embodiment, the wick must conduct the full charge that is supplied to the electrode. The liquid provides a means of conducting the charge from the center of the wick to the outer surfaces where vaporization takes place in the same manner as with wicks that are conductive, although a higher voltage is required to generate the same amount of vapors.

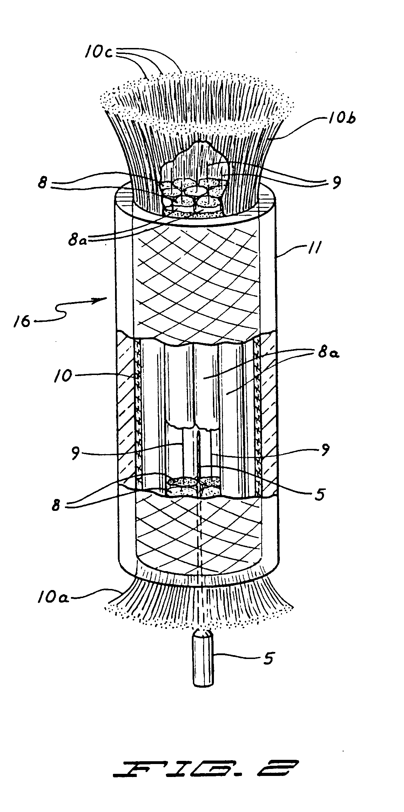

Fig.2 details a preferred design of a wick or emitter assembly 16. Wick assembly 16 is comprised of a center electrode assembly which is made of ceramic fibers 8 in which are embedded stainless steel wires 9. Fibers 8 are preferably formed into a plurality of elongated capillament bundles 8a as shown. The fibers 8 may be braided or twisted, with wires 9 either extending straight therein or intertwined with the fibers. This core is covered by a glass fiber braid in the form of a sleeve 10 preferably comprised of a plurality of separate capillament bundles of fine fibers or filaments which are exposed at 10b at the top of the assembly. An outer cover of glass tubing 11 may also be provided if the sleeve 10 does not provide a sufficiently strong, liquid impervious outer layer. In this design the inner conductive fiber core contacts the electrode 5, which preferably extends at least partially into the inner core of capillaments 8a as shown. It also holds the liquid that is transferred to the glass fiber braid. The core wires 9 help shape the electric field which in turn affects the vapor-aerosol pattern and also the air ion output. The outer glass fiber braid 10 moves the liquid by capillary action from bottom fibers 10a to the top through exposed top fibers 10b where the electrostatic field breaks down the surface tension of the liquid, and from the very tips 10c of the glass fibers the liquid is converted to vapor and/or aerosols and released. Capillament bundles 8a also assist in moving the liquid through the assembly 16 by wicking or capillary action.

This design also is a very effective air ion emitter. This illustration is an example of the concept of using a number of materials which together have the desired properties of porosity, conductance, and capillary action, and will generate vapor and/or aerosols when electrified by a voltage high enough to break the surface tension of the desired liquid.

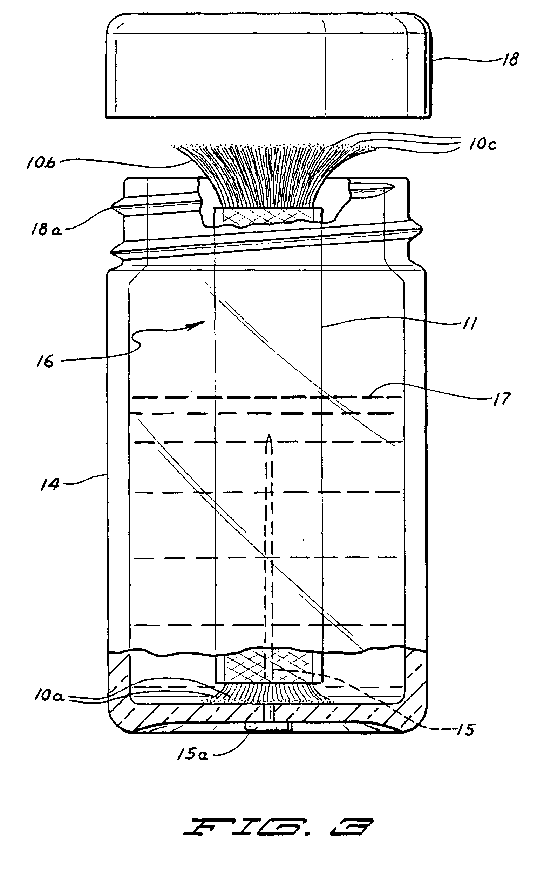

Fig. 3 is a preferred embodiment of a device that will also provide a means of containing the liquid that would be supplied to the wick assembly 16 of Fig. 2. In this embodiment a glass bottle 14 contains the wick assembly 16 and has a high voltage electrode 15 which extends through the bottom of the bottle. It also has a contact terminal 15a on the bottom of the bottle in order to provide a means of supplying a charge to the wick assembly 16. The desired liquid 17 is contained within the bottle and is continuously moved to the top of the wick by capillary action. That action is enhanced by the extension of fiber end segments 10a into the liquid at the lower end of wick assembly 16. The bottle can be sealed by a cap 18 and stored for later use without loss of liquid due to evaporation or spilling. This embodiment is a self-contained system that will generate vapor and/or aerosols and also air ions when it is provided a high voltage DC signal to the base electrode and the bottle cap 18 is removed. A threaded cap 18 may be used for attachment to threads 18a on bottle 14.

The embodiment shown inFig.4 is a device that holds the vaporizing bottles detailed in Fig. 3. The device is comprised of an insulating support 19 made of porcelain, glass, plastic, or similar material. A high voltage DC signal is supplied to the contact terminal connector 20 by a high voltage wire 2. The electrode contact 15a of the bottle 14 makes contact with the power contact 20 as shown. This provides power to the wick assembly 16 and causes the vapor and/or aerosols to emanate from the top of the bottle into the air. The bottle 14 is secured within the recess 21 of the support column.

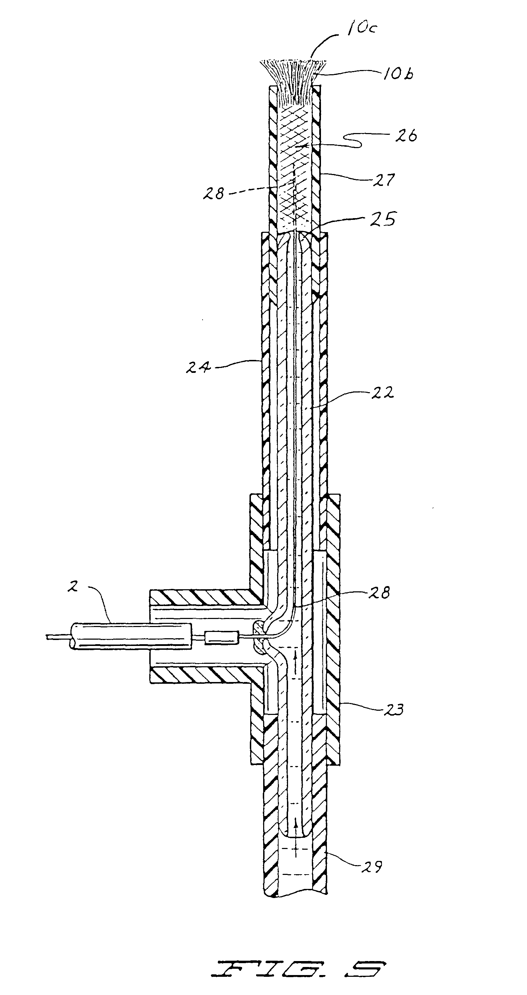

Fig. 5 is an illustration of an embodiment of a "vaporizing emitter." The emitter is comprised of the following: a glass capillary tube 22 fitted securely within a modified fluid TEE connector 23, and a polycarbonate tube 24 which serves to protect the glass capillary tube and also couples the electrostatic wick assembly 26 to the tip 25 of the capillary tube 22. In this embodiment, the glass tube 11 from Fig. 2 is replaced with a larger tapered fluoroplastic tube 27 which supports and protects the "wick" assembly 26. Otherwise, the wick assembly may be the same as that shown at 16 in Fig. 2. The high voltage is supplied through wire conduit 2 to a Nichrome wire 28 which is inserted into the capillary tube 22 and makes contact with the center core of the wick assembly 26 at the tip of the glass capillary tube. In this design liquid is supplied to the glass capillary 22 through a tube 29 from a fluid control system which meters the desired amount of liquid to the vaporizing emitter assembly. A suitable liquid pump connected to a supply source of desired liquid may be used to provide the metered flow of liquid. The liquid may be an aromatic essence, deodorant, disinfectant, fumigant, fungicide, insecticide or bactericide.

A moving air steam may be used to remove the generated vapor. In this design the vapor control is achieved by controlling the liquid feed rate, the high voltage, and the air stream velocity.

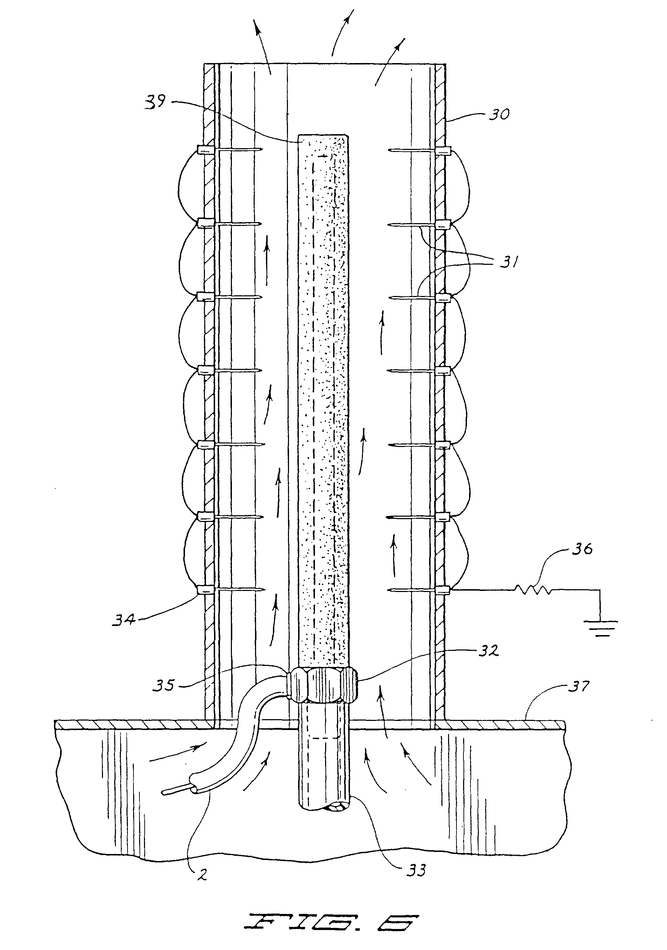

Fig. 6 is an illustration of a vaporizing emitter which is outside the scope of the invention as defined in the appended claims because it is based upon the use of a porous metal tube. It will, nevertheless, be described because, as indicated below, the porous metal tube may be replaced by a wick assembly 16 in accordance with the appended claims. The tube 39 is surrounded by a control grid cylindrical enclosure tube 30 which contains a plurality of needles 31 positioned around the circumference and also along the length of the porous metal emitter tube 39. These needles or points are connected together electrically and may be powered by a DC or AC signal provided at 34 or connected to ground through a resistor 36 or directly. The porous metal tube 39 is configured to be supplied with the desired fluid by means of a fluid connection 32 and a fluid feed line 33. The fluid feed rate is controlled by automatic or manual means. The high voltage DC signal 2 is supplied to the porous tube by a contact ring 35 on connector fitting 32 in order to provide the electrostatic charge to the porous metal tube. The position of the plurality of needles near this tube causes the formation of a high concentration of electric field lines, and as a result there is a breakdown of the vapor pressure of the liquid which has saturated the metal tube. The area under each needle becomes an "active region" of vapor and/or aerosol generation. The generated vapor and/or aerosols are removed from the confinement of the enclosure tube by some means of air flow through the tube.

By controlling the voltage and the liquid feed rate supplied to the porous emitter tube, and the signal or ground applied to the needle array, and the air flow rate through the enclosure tube 30, it is possible to control the aerosol and/or vapor generation output of the device.

In an embodiment according to the present invention, the wick assembly 16 of Fig. 2 is substitued for the porous metal tube 39 as the vapor or aerosol emitter inside of air passage, enclosure tube 30. In such an embodiment, the secondary electrodes in the form of needles 31 would not be required and would not be used. As with the embodiment of Fig. 5 described above, a desired liquid would be supplied in metered amounts, as from a pump, to supply line 33, and thence into the wick assembly 16, and a moving air stream would be used to remove the generated vapor. For that purpose, a blower (not shown) is provided in housing 37 to provide a pressurized stream of air through tube 30 and over the vapor emitter assembly. Any of the liquids described above may be supplied through supply line 33, and the generated vapors may be released directly into a room or into the duct work or related air distribution system of a building air conditioning or ventilation system. For the latter applications, the upper, outlet end of air flow enclosure tube 30 could be connected to an air duct, or the wick assembly emitter could be mounted directly inside a duct, with air flow tube 30 comprising such a duct. The electrostatically charged vapor or aerosol may be thus injected into the air handling system of a building to odorize by the use of essential oils or perfumes or to disinfect by the use of fungicides, bactericides, fumigants, insecticides, disinfectants and the like. In this manner, micro-organisms such as bacteria, fungus, mold and the like which collect in air conditioning systems and particularly on the surfaces of ducts and air handling equipment may be treated by such electrostatically charged vapors and controlled.

In summary, the embodiments which have been described above serve to illustrate a novel method of being able to generate liquid based aerosols, vapors and also air ions with a variety of means of control over the quantity of vapor and/or aerosols, the size of the aerosols and also air ions. The fundamental base of these methods, devices, and apparatus is based upon the use of electrostatic charges being applied to a preferably semi-conductive, wick-like, porous, capillament assembly comprising a plurality of fiber filaments arranged in a tubular configuration which terminate at the dispensing end in a plurality of exposed, freely extending dispensing tips, which is also supplied with the desired liquid which is to be vaporized. In addition, the placement of a control grid assembly within or near the aerosol/vapor generation zone will provide a means of affecting the electric field concentration and pattern thereby also having an influence upon the aerosol/vapor/air ion generation.

## Claims

1. Apparatus for generating electrostatically charged aerosols and/or vapors comprising:
a porous, capillary unit (10) of elongated configuration having a dispensing end, said capillary unit being comprised of a plurality of fiber filaments (10a, 10b) arranged in a tubular configuration which terminate at said dispensing end in a plurality of exposed, freely extending dispensing tips (10c) ;
electrode means, said electrode means comprising an electrode (5,15) in contact with said capillary unit;
electrical contact means (15a) on said electrode for connecting said electrode to a source (1) of high voltage, direct current (DC) power; and
means (8,14) for supplying liquid to said capillary unit, whereby liquid can pass through said porous capillary unit in proximity to said electrode and be electrostatically charged and dispensed as aerosols and/or vapors from said freely extending tips of said capillary unit.

2. Apparatus as defined in claim 1 wherein:
said fiber filaments (10a, 10b) are braided into capillament bundles, the extremities (10c) of which at said dispensing end of said capillary unit (10) are open and unbraided to provide said freely extending, filament tip extremities (10c).

3. Apparatus as defined in claim 1 or claim 2 wherein:
said capillary unit (10) further comprises a central, elongated core made up of a plurality of semi-conductive capillaments (8a) through which liquid can pass, with said central core being positioned inside of said tubular configuration of fiber filaments (10a, 10b) in electrically conductive relation with said electrode (5,15).

4. Apparatus as defined in claim 3 wherein:
said capillary unit (10) is contained and protected in an outer, liquid impervious tubular member (11).

5. Apparatus as defined in claim 3 or claim 4 wherein:
said capillaments (8a) each comprise a bundle of fibers (8) in which a wire conductor (9) is embedded.

6. Apparatus as defined in claim 5 wherein:
said electrode (5,15) is elongated and extends at least partially into said core substantially centrally thereof.

7. Apparatus as defined in any one of claims 1 to 6 wherein:
said capillary unit (10) is contained within an outer, liquid impervious vessel (14) capable of holding a supply of liquid and comprising said means for supplying liquid to said capillary unit.

8. Apparatus as defined in claim 7 wherein:
said fiber filaments (10a, 10b) terminate at their lower ends in end segments (10a) which extend into the vessel (14) and, in use, into liquid present therein.

9. Apparatus as defined in claim 7 or claim 8 and further including:
an electrically insulating holder (19) having an upwardly opening recess (21) within which said vessel (14) is removably received for dispensing operation, said holder having electrical connector means (20) therein for connecting said electrical contact means (15a) on said electrode (15)to a power supply wire (2).

10. Apparatus as defined in any one of claims 1 to 6 and further including:
a tubular passage (30) within which said capillary unit (10) is positioned;
said means for supplying liquid comprises a liquid supply line (33) connected to said capillary unit; and means for delivering a pressurized stream of air into said tubular passage for flow over said capillary unit and removal of aerosol and/or vapors emitted from said capillary unit.

11. Apparatus as defined in claim 10 wherein:
said means for delivering a pressurized stream of air comprises a blower housing (37) connected to one end of said tubular passage (30).

12. The use of apparatus as defined in any one of claims 1 to 11 to generate electrostatically charged aerosols and/or vapors and/or air ions.

13. The use of apparatus as defined in claim 11 to enhance the air quality within a building by supplying an aromatic essence, deodorant, disinfectant, fumigant, fungicide, insecticide or bactericide substance as said liquid, wherein the opposite end of said tubular passage (30) is connected to a duct of an air handling system of a building.

14. A method of generating air quality enhancing vapors and/or aerosols and dispensing them into the air handling system of a building comprising:
placing an emitter having a dispensing end (10c) in air flow communication with the air handling system of a building, said emitter comprising;
a plurality of fiber filaments (10a, 10b) arranged in a tubular configuration which terminate at said dispensing end in a plurality of exposed, freely extending dispensing tips (10c) ;
liquid passage means (22);
means (29,32,33) for supplying liquid to said liquid passage means;
means (2,28,35) for imparting an electrostatic charge to liquid in said emitter; and
air flow passage means (30) in fluid flow juxtaposition to said liquid passage means (22);
directing a pressurized flow of liquid to said liquid passage means (22) from said means (29,32,33) for supplying liquid, said liquid being selected from the group of air quality enhancing substances comprising aromatic essences, deodorants, disinfectants, fumigants, fungicides, insecticides, and bactericides;
applying an electrostatic charge to liquid in said emitter and thereby generating vapors and/or aerosols from said freely extending tips (10c); and,
passing a stream of pressurized air through said air flow passage means (30) and thereby picking up said electrostatically charged vapors and/or aerosols in said air stream; and thence directing said stream of pressurized air into said air handling system.

## Patentansprüche

1. Vorrichtung zum Erzeugen elektrostatisch geladener Aerosole und/oder Dämpfe, umfassend:
eine poröse Kapillareinheit (10) mit langgestreckter Konfiguration, die ein Abgabeende besitzt, wobei die Kapillareinheit mehrere Faserfäden (10a, 10b) umfaßt, die in einer rohrförmigen Konfiguration angeordnet sind und am Abgabeende in mehreren freiliegenden, sich frei erstreckenden Abgabespitzen (10c) enden;
eine Elektrodeneinrichtung, die eine Elektrode (5, 15) enthält, die mit der Kapillareinheit in Kontakt ist;
eine elektrische Kontakteinrichtung (15a), die sich an der Elektrode befindet und die Elektrode mit einer Quelle (1) für Hochspannungs-Gleichstromleistung (Hochspannungs-DC-Leistung) verbindet; und
eine Einrichtung (8, 14), die an die Kapillareinheit Flüssigkeit liefert, wobei sich die Flüssigkeit durch die poröse Kapillareinheit in der Nähe der Elektrode bewegen und elektrostatisch aufgeladen werden kann und in Form von Aerosolen und/oder Dämpfen von den frei sich erstreckenden Spitzen der Kapillareinheit abgegeben werden kann.

2. Vorrichtung nach Anspruch 1, wobei:
die Faserfäden (10a, 10b) zu Kapillarelementbündeln geflochten sind, deren Enden (10c) am Abgabeende der Kapillareinheit (10) offen und nicht geflochten sind, um die frei sich erstreckenden Fadenspitzen-Enden (10c) zu schaffen.

3. Vorrichtung nach Anspruch 1 oder Anspruch 2, wobei:
die Kapillareinheit (10) ferner einen zentralen, langgestreckten Kern umfaßt, der aus mehreren halbleitenden Kapillarelementen (8a) gebildet ist, durch die sich Flüssigkeit bewegen kann, wobei der zentrale Kern in der rohrförmigen Konfiguration der Faserfäden (10a, 10b) in elektrisch leitender Beziehung mit der Elektrode (5, 15) angeordnet ist.

4. Vorrichtung nach Anspruch 3, wobei
die Kapillareinheit (10) in einem äußeren, flüssigkeitsundurchlässigen röhrenförmigen Element (11) enthalten und geschützt ist.

5. Vorrichtung nach Anspruch 3 oder Anspruch 4, wobei:
die Kapillarelemente (8a) jeweils ein Faserbündel (8) umfassen, in das ein Drahtleiter (9) eingebettet ist.

6. Vorrichtung nach Anspruch 5, wobei:
die Elektrode (5, 15) langgestreckt ist und sich wenigstens teilweise und zentral in den Kern erstreckt.

7. Vorrichtung nach einem der Ansprüche 1 bis 6, wobei:
die Kapillareinheit (10) in einem äußeren, flüssigkeitsundurchlässigen Gefäß (14) enthalten ist, das zugeführte Flüssigkeit halten kann und die Einrichtung zum Liefern von Flüssigkeit an die Kapillareinheit umfaßt.

8. Vorrichtung nach Anspruch 7, wobei:
die Faserfäden (10a, 10b) an ihren unteren Enden in Endsegmenten (10a) enden, die sich in das Gefäß (14) und im Gebrauch in die darin vorhandene Flüssigkeit erstrecken.

9. Vorrichtung nach Anspruch 7 oder Anspruch 8 und ferner umfassend:
eine elektrisch isolierende Halterung (19) mit einer nach oben geöffneten Aussparung (21), in der das Gefäß (14) für die Abgabeoperation entnehmbar aufgenommen ist, wobei die Halterung eine elektrische Verbindereinrichtung (20) besitzt, um die elektrische Kontakteinrichtung (15a) an der Elektrode (15) mit einem Leistungsversorgungsdraht (2) zu verbinden.

10. Vorrichtung nach einem der Ansprüche 1 bis 6 und ferner umfassend:
einen röhrenförmigen Durchlaß (30), in dem die Kapillareinheit (10) angeordnet ist;
wobei die Einrichtung zum Liefern von Flüssigkeit eine Flüssigkeitsversorgungsleitung (33) aufweist, die mit der Kapillareinheit verbunden ist; und eine Einrichtung zum Liefern eines mit Druck beaufschlagten Luftstroms in den röhrenförmigen Durchlaß, so daß er über die Kapillareinheit strömt und die Aerosole und/oder Dämpfe, die von der Kapillareinheit abgegeben werden, entfernen kann.

11. Vorrichtung nach Anspruch 10, wobei:
die Einrichtung zum Liefern einer mit Druck beaufschlagten Luftströmung ein mit einem Ende des röhrenförmigen Durchlasses (30) verbundenes Gebläsegehäuse (37) umfaßt.

12. Verwendung der Vorrichtung nach einem der Ansprüche 1 bis 11, um elektrostatisch aufgeladene Aerosole und/oder Dämpfe und/oder Luftionen zu erzeugen.

13. Verwendung der Vorrichtung nach Anspruch 11, um die Luftqualität in einem Gebäude durch Zuführen einer Duftessenz-, Deodorant-, Desinfektions-, Räucher-, Fungizid-, Insektizid- oder Bakterizid-Substanz als Flüssigkeit zu verbessern, wobei das gegenüberliegende Ende des röhrenförmigen Durchlasses (30) mit einem Röhrensystem eines Belüftungssystems eines Gebäudes verbunden ist.

14. Verfahren zum Erzeugen von die Luftqualität verbessernden Dämpfen und/oder Aerosolen und zum Abgeben der Dämpfe und/oder Aerosole in das Belüftungssystem eines Gebäudes, umfassend:
Anordnen eines Spenders mit einem Abgabeende (10c) in einer Luftströmungsverbindung mit dem Belüftungssystem eines Gebäudes, wobei der Spender umfaßt:
mehrere Faserfäden (10a, 10b), die in einer röhrenförmigen Konfiguration angeordnet sind und am Abgabeende in mehreren freiliegenden, sich frei erstreckenden Abgabespitzen (10c) enden;
eine Flüssigkeitsdurchlaßeinrichtung (22);
eine Einrichtung (29, 32, 33) zum Liefern von Flüssigkeit an die Flüssigkeitsdurchlaßeinrichtung;
eine Einrichtung (2, 28, 35) zum Beaufschlagen der Flüssigkeit mit elektrostatischer Ladung im Spender; und
eine Luftströmungsdurchlaßeinrichtung (30), deren Fluidströmung diejenige der Flüssigkeitsdurchlaßeinrichtung (22) berührt;
Richten einer mit Druck beaufschlagten Flüssigkeitsströmung auf die Flüssigkeitsdurchlaßeinrichtung (22) von der Einrichtung (29, 32, 33) zum Liefern von Flüssigkeit, wobei die Flüssigkeit aus der Gruppe von die Luftqualität verbessernden Substanzen gewählt ist, die Duftessenzen, Deodoranzien, Desinfektionsmittel, Räuchermittel, Fungizide, Insektizide und Bakterizide umfaßt;
Beaufschlagen der Flüssigkeit mit elektrostatischer Ladung im Spender und dadurch Erzeugen von Dämpfen und/oder Aerosolen aus den frei sich erstreckenden Spitzen (10c); und
Schicken eines Stroms von mit Druck beaufschlagter Luft durch die Luftströmungsdurchlaßeinrichtung (30) und dadurch Aufnehmen der elektrostatisch aufgeladenen Dämpfe und/oder Aerosole im Luftstrom; und dann Leiten des Stroms von mit Druck beaufschlagter Luft in das Belüftungssystem.

## Revendications

1. Appareil pour engendrer des aérosols et/ou des vapeurs chargés électrostatiquement, comprenant :
une unité capillaire poreuse (10) de configuration allongée comportant une extrémité distributrice, l'unité capillaire étant constituée d'une pluralité de filaments de fibre (10a, 10b) disposés selon une configuration tubulaire se terminant à l'extrémité distributrice en une pluralité de pointes distributrices accessibles s'étendant librement,
des moyens d'électrode, les moyens d'électrode comportant une électrode (5, 15) en contact avec l'unité capillaire,
des moyens de contact électrique (15a) sur l'électrode pour connecter l'électrode à une source d'alimentation (1) de courant continu (DC) à haute tension, et
des moyens (8,14) de fourniture de liquide à l'unité capillaire, permettant le passage du liquide à travers l'unité capillaire poreuse à proximité de l'électrode, la charge électrostatique et la distribution de celui-ci, sous forme d'aérosols et/ou de vapeurs, depuis les pointes s'étendant librement de l'unité capillaire.

2. Appareil selon la revendication 1, dans lequel les filaments de fibre (10a, 10b) sont guipés en faisceaux de capillaires dont les extrémités (10c), à l'extrémité de distribution de l'unité capillaire (10), sont ouvertes et déguipées pour fournir les extrémités à pointe de filament s'étendant librement (10c).

3. Appareil selon l'une des revendications 1 et 2, dans lequel l'unité capillaire (10) comporte en outre un noyau central allongé constitué d'une pluralité de capillaires semi-conducteurs (8a) qu'un liquide peut traverser, le noyau central étant disposé à l'intérieur de la configuration tubulaire de filaments de fibre (10a, 10b) en relation de conduction électrique avec l'électrode (5, 15).

4. Appareil selon la revendication 3, dans lequel l'unité capillaire (10) est contenue dans un élément tubulaire externe (11) imperméable aux liquides, qui la protège.

5. Appareil selon l'une des revendications 3 et 4, dans lequel les capillaires (8a) comportent chacun un faisceau de fibres (8) dans lequel est intégré un fil conducteur (9).

6. Appareil selon la revendication 5, dans lequel l'électrode (5, 15) est allongée et s'étend au moins partiellement dans le noyau, sensiblement de façon centrale.

7. Appareil selon l'une des revendications 1 à 6, dans lequel l'unité capillaire (10) est contenue dans un récipient externe (14) imperméable au liquide agencé pour contenir un liquide d'alimentation et comportant les moyens de fourniture de liquide à l'unité capillaire.

8. Appareil selon la revendication 7 , dans lequel les filaments de fibre (10a, 10b) se terminent à leurs extrémités inférieures par des tronçons d'extrémité (10a) qui s'étendent dans le récipient (14) et, en utilisation, dans du liquide qui s'y trouve.

9. Appareil selon l'une des revendications 7 et 8, comportant en outre un support isolant électrique (19) comportant une cavité (21) ouverte vers le haut dans laquelle le récipient (14) est reçu de façon amovible pour le fonctionnement de distribution, le support comprenant des moyens de connecteur électrique (20) pour connecter les moyens de contact électrique (15a) de l'électrode (15) à un fil d'alimentation électrique (2).

10. Appareil selon l'une des revendications 1 à 6, comportant en outre un passage tubulaire (30) dans lequel est placée l'unité capillaire (10), les moyens de fourniture de liquide comprenant une liaison de fourniture de liquide (33) reliée à l'unité capillaire, et il est prévu des moyens de distribution d'un flux d'air sous pression dans le passage tubulaire pour qu'il circule sur l'unité capillaire et enlève l'aérosol et/ou les vapeurs émis de l'unité capillaire.

11. Appareil selon la revendication 10, dans lequel les moyens de distribution d'un flux d'air sous pression comprennent un carter de soufflante (37) relié à une extrémité du passage tubulaire (30).

12. Utilisation de l'appareil selon l'une des revendications 1 à 11 pour engendrer des aérosols et/ou vapeurs chargés électrostatiquement et/ou des ions dans l'air.

13. Utilisation de l'appareil selon la revendication 11 pour améliorer la qualité de l'air dans un immeuble en fournissant ledit liquide sous forme d'une substance à essence aromatique, désodorisante, désinfectante, fumigante, fongicide, insecticide ou bactéricide, l'extrémité opposée du passage tubulaire (30) étant reliée à un tuyau d'un système de traitement de l'air d'un immeuble.

14. Procédé pour engendrer des vapeurs et/ou des aérosols d'amélioration de la qualité de l'air et pour les distribuer dans le système de traitement de l'air d'un immeuble, comprenant les étapes suivantes :
- on met un émetteur, comportant une extrémité de distribution (10c), en communication de flux d'air avec le système de traitement de l'air d'un immeuble, l'émetteur comprenant :
une pluralité de filaments de fibre (10a, 10b) disposés en configuration tubulaire se terminant à l'extrémité de distribution en une pluralité de pointes distributrices accessibles s'étendant librement (10c),
des moyens de passage de liquide (22),
des moyens (29, 32, 33) de fourniture de liquide aux moyens de passage de liquide,
des moyens (2, 28, 35) pour appliquer une charge électrostatique au liquide contenu dans l'émetteur, et
des moyens de passage de flux d'air (30) juxtaposés, en ce qui concerne le flux de fluide, avec les moyens de passage de liquide (22),
- on dirige un flux de liquide sous pression dans les moyens de passage de liquide (22) depuis lesdits moyens (29, 32, 33) de fourniture du liquide, le liquide étant choisi dans le groupe de substances d'amélioration de la qualité de l'air comprenant les essences aromatiques, les déodorants, les désinfectants, les fumigants, les fongicides, les insecticides et les bactéricides,
- on applique une charge électrostatique au liquide contenu dans l'émetteur et on engendre ainsi des vapeurs et/ou aérosols sortant par les pointes s'étendant librement (10c), et
- on fait passer un flux d'air sous pression à travers les moyens de passage de flux d'air (30) et ainsi on capture les vapeurs et/ou aérosols chargés électrostatiquement dans le flux d'air, puis on dirige le flux d'air sous pression dans le système de traitement de l'air.
